# EUROPEAN PATENT SPECIFICATION

(11) **EP 1 563 847 B1**
(45) Date of publication and mention of the grant of the patent: **16.12.2009**
(21) Application number: 03756720.3
(22) Date of filing: 21.10.2003
(51) Int. Cl.: A61K 45/00, A61K 38/17, A61P 35/00, A61P 37/04

(54) **AGENT ELEVATING DENDRITIC CELL PRECURSOR LEVEL IN BLOOD**
WIRKSTOFF ZUR ERHÖHUNG DER ANZAHL AN DENDRITISCHEN VORLÄUFERZELLEN IM BLUT
AGENT ELEVATEUR DU NIVEAU DE PRECURSEUR DE CELLULES DENDRITIQUES DANS LE SANG

(30) Priority: 24.10.2002 JP 2002310090; 13.06.2003 JP 2003170091
(43) Date of publication of application: 17.08.2005
(73) Proprietor: ECI, Inc., Meguro-ku Tokyo 153-0042 (JP); MATSUSHIMA, Kouji, Matsudo-shi, Chiba 271-0092 (JP)
(72) Inventor: MATSUSHIMA, Kouji, Matsudo-shi, Chiba 271-0092 (JP); YONEYAMA, Hiroyuki, Bunkyo-ku, Tokyo 112-0002 (JP); ZHANG, Yuang, Shanghai 200092 (CN); KANEGASAKI, Shiro, Meguro-ku, Tokyo 153-0041 (JP); AKUTA, Teruo, Kumamoto-shi, Kumamoto 862-0901 (JP)
(74) Representative: Vossius & Partner
(86) International application number: PCT/JP2003/013416
(87) International publication number: WO 2004/037288

(56) References cited:
- EP-A- 1 013 276
- WO-A-00/47228
- WO-A-99/11666
- WO-A-2004/024088
- WO-A1-01/83548
- BROXMEYER H E ET AL: "Myeloid progenitor cell proliferation and mobilization effects of BB10010, a genetically engineered variant of human macrophage inflammatory protein-1alpha, in a phase I clinical trial in patients with relapsed/refractory breast cancer." BLOOD CELLS, MOLECULES & DISEASES MAR 1998, vol. 24, no. 1, March 1998 (1998-03), pages 14-30, XP001011408 ISSN: 1079-9796
- DIEU, MARIE-CAROLINE ET AL.: 'Selective recruitment of immature and mature dendritic cells by distinct chemokines expressed in different enatomic sites' J. EXP. MED. vol. 188, no. 2, 1998, pages 373 - 386, XP002121899
- LIN, CHEN-LUNG ET AL.: 'Macrophage-tropic HIV induces and exploits dendric cell chemotaxis' J. EXP. MED. vol. 192, no. 4, 2000, pages 587 - 593, XP002974853
- HUNTER, M.G. ET AL.: 'BB-10010: an active variant of human macrophage inflammatory protein-1a with improved pharmaceutical properties' BLOOD vol. 86, no. 12, 1995, pages 4400 - 4408, XP000611695

## Description

### Technical Field

This invention relates to an agent for elevating dendritic cell level in the blood and a novel agonist derivative chemically modified with an amphipathic polymer which has the above effect.

### Background Art

It is known that dendritic cells (hereinafter sometimes abbreviated as DCs), which are cells derived from CD34⁺ cells (i.e., stem cells), morphologically characterized by having dendritic spines and widely distributed in the living body, efficiently uptake and present an antigen to T cells owing to the trafficking (migration) ability *in vivo.* Namely, dendritic cells are derived from bone marrow stem cells and participate in immunological surveillance while altering tissues or organs in which they are distributed in the course of proliferation, differentiation and maturation, similar to other immunocompetent cells.

The proliferation, differentiation and maturation process of dendritic cells can be divided into the following 5 stages. (1) DC progenitors which exist mainly in the bone marrow and produce precursors while undergoing self-replication. (2) DC precursors which are constantly supplied in the steady state from the bone marrow into organs via blood circulation in the living body. Then these precursors pass through the vascular wall and get into tissues. Thus, they are distributed in the epithelium and the interstitium. (3) Sentinel DCs which acquire phagocytic ability and uptake an antigen invading an organ in which they are distributed. Typical examples thereof include epidermal Langerhans cells, dendritic cells in the respiratory epithelium, interstitial DCs in parenchymatous organs such as the heart and the liver, and so on. (4) Antigen-transporting DCs which have matured one stage further due to the uptake of antigen data and acquire migration ability. In human tonsil, dendritic cells migrate across the tonsil tissue from the crypt epithelium to the T cell area (the interfollicular area) and induce an immune response therein. In other organs, many dendritic cells enter into afferent lymphatic vessels, then lymphogenously migrate toward so-called lymph nodes and accumulate in the T cell area (the paracorteal area). (5) Mature (antigen-presenting) DCs which have matured to attain the final stage in lymph organs. Then these dendritic cells form a cell cluster together with T cells, and select antigen-specific T cells followed by antigen-presentation. Next, the dendritic cells seemingly die by apoptosis in the T cell area *(*Igaku no Ayumi, Vol. 200, No. 6, pp.472-476 (2002)).

It is known that immunotherapy for a disease can be potentiated by proliferating dendritic cells (precursors) collected from the peripheral blood *in vitro* in the presence of, for example, GM-CSF and TNFα, then stimulating them with a disease antigen such as tumor cells and then returning them into the living body. Attempts have been made to apply this treatment, which is called immunotherapy or vaccine therapy with the use of dendritic cells, to various diseases such as melanoma, kidney caner, prostate cancer, leukemia and metastatic malignant tumor. That is to say, use of dendritic cells for therapeutic purposes have attracted public attention and the immunotherapy with the use of dendritic cells is employed not only in treating tumor but also in the fields of infections, transplantation and autoimmune (see, for example, Kayo Inaba, Saibo Kogaku, Vol.19, No.9, 1282-1286 (2000); Hiroshi Kawamoto et al., Saibo Kogaku, Vol. 19, No.9, 1289-1293 (2000); Tomonori Iyoda et al., Saibo Kogaku, Vol.19, No.9, 1311-1317 (2000); and Takuya Takayama et al., Bunshi Saibo Chiryo, Vol.2, No.6, 53-56 (2001)).

As receptors expressed in immature dendritic cells, there are known CCR1, CCR2, CCR4, CCR5, CCR6, CCR11 and CXCR4. As ligands for these receptors, there are known MIP-1α, MIP-1β, RANTES, MARC, LCC-1(ref), MCP-1, MCP-2, MCP-3, MCP-4, MCP-5, TARC, MDC, MIP-3α, MIP-3β, LARC, TECK, BLC, SDF-1, Exodus-1, Exodus-2 and so on. Among all, it is known that MIP-1α, RANTES, MARC and LCC-1 (ref) are ligands commonly for CCR-1 and CCR-5 (see, for example, Hideki Nakano, Saibo Kogaku, Vol.19, No.9, 1304-1310 (2000)).

It is also known that there are functional derivatives of ligands (agonists) which are comparable in biological activity to the ligands. In the case of MIP-1α, for example, an MIP-1α mutant (BB10010) in which Asp at the 26-position in MIP-1α is substituted by Ala and which is composed of 69 amino acids starting with Ser at the amino end is known. It is found out that this MIP-1α mutant has a remarkably improved anticoagulant ability and an activity comparable to the wild type. Thus, there is a plan currently under discussion for improving granulocytopenia, which occurs as a side effect of chemotherapy for cancer, by using this mutant (E. Marshall et al., European Journal of Cancer, Vol.34, No.7, pp.1023-1029 (1998)).

It has been already known that neocarzinostatin chemically modified with a partially butyl-esterified styrene-maleic acid copolymer, which is an amphipathic polymer, is usable as a carcinostatic agent (general name: zinostatin stimalamer, Japanese Patent Publication of Examined Application 1-33119). It is also known that, when administered into the blood, this drug accumulates almost selectively in solid tumor and is sustained therein over a long period of time, i.e., showing a so-called EPR effect. Owing to these characteristics, it has been employed as a carcinostatic agent specifically targeting cancer. Also, a peptidic agonist chemically modified with an amphipathic polymer and its functional derivative are known (WO 01/83548) . Moreover, it is known that xanthine oxidase modified with polyethylene glycol shows the EPR effect on tumor cells (Japanese Patent Publication of Unexamined Application 11-060499). In each of these cases, an antitumor effect is established by using a substance directly attacking against tumor cells. Namely, these methods aim at accumulating an aggressive substance selectively in a target site to thereby minimize the effects of the substance on normal cells or tissues.

It is also known that a protein modified with a polyethylene glycol derivative which is an amphipathic polymer exhibits delayed clearance or lowered antigenicity *in vivo* (Yoshimoto et al., Jpn. J. Cancer Res., 77, 1264 (1986); Abuchowski et al., Cancer Biochem. Biophys., 7, 175 (1984); Japanese Patent Publication of Unexamined Application 61-178926; Japanese Patent Publication of Unexamined Application 62-115820; Domestic Re-publication No. of PCT international publication for patent application WO96/28475; Publication No. of Japanese translations of PCT international publication for patent application 10-513187; Japanese Patent Publication of Unexamined Application 11-310600, Publication No. of Japanese translations of PCT international publication for patent application 2000-517304 ). Furthermore, there are known interleukin-1, interleukin-6, interferon and so on each modified with polyethylene glycol (Japanese Patent Publication of Unexamined Application 5-117300; Japanese Patent Publication of Unexamined Application 6-256394; and Japanese Patent Publication of Unexamined Application 9-25298).

### Disclosure of the Invention

As described above, the fields of therapeutic applications of dendritic cells are now expanding. However, dendritic cell precursors exist only in a small amount in the peripheral blood and, therefore, it is still difficult to obtain them in a therapeutically useful amount even though they are proliferated *in vitro.* Therefore, it is a key point in performing a therapy with the use of dendritic cells in practice to elevate dendritic cell precursor level in the peripheral blood of a patient.

The present invention aims at providing a technical means therefor.

The present inventor previously found out that dendritic cell precursors (i.e., F4/80 antigen-negative, B220 antigen-negative and CD11c antigen-positive cells) were recruited into the blood by administering dead *Propionicbacterium acnes* (hereinafter abbreviated as *P*. *acnes)* cells to mice. As the results of the subsequent studies, it was clarified that the recruitment of dendritic cell precursors is inhibited by macrophage inflammatory protein-1α (MIP-1α) antibody and, therefore, it was assumed that MIP-1α and, in its turn, agonists to receptors expressed in immature dendritic cells might participate in the recruitment mechanism of dendritic cell precursors. By further conducting studies, it was unexpectedly found out that the exogenous administration of such an agonists (for example, MIP-1α) results in an increase in the concentration level of dendritic cell precursors in the blood at least several ten-fold, thereby completing the present invention. Unless otherwise noted, the term "dendritic cell" is employed herein as a generic term referring to dendritic cells at various stages of the maturation.

However, there frequently arises a problem that many ligands such as natural MIP-1α form aggregates and undergo sedimentation under physiological conditions, thereby losing the activity. To overcome this problem, studies were further conducted so as to enhance the effect of these ligands of elevating dendritic cell precursor concentration in the blood and improve the stability thereof in the blood.

Namely, the present inventors paid attentions to MIP-1α as a typical example of agonists to receptors expressed in immature dendritic cells and BB10010 which is one of functional derivatives of MIP-1α, and attempted to chemically modify these ligands with an amphipathic polymer. As a result, they have found out that a chemically modified ligand suffers from no damage in its activity but, on the contrary, shows an improvement in the activity, thereby successfully finding a novel agonist derivative.

Accordingly, the present invention relates to: a pharmaceutical composition comprising BB-10010 which is chemically modified with a partially butyl-esterified styrene-maleic copolymer as the active ingredient. Furthermore, the invention also relates to the use of BB-10010, which is chemically modified with a partially butyl-esterified styrene-maleic copolymer, for the preparation of a pharmaceutical composition for treating a solid tumor as well as to BB-10010, which is chemically modified with a partially butyl-esterified styrene-maleic copolymer. Also described herein is: (1) an agent for elevating dendritic cell precursor level in the blood which comprises an agonist to a receptor expressed in immature dendritic cells or a functional derivative thereof as the active ingredient; and (2) an agent for elevating dendritic cell precursor level in the blood which comprises an agonist to receptor CCR1 or CCR5 or a functional derivative thereof as the active ingredient.

More specifically speaking, here described is: (3) an agent for elevating dendritic cell precursor level in the blood wherein the agonist is selected form among MIP-1α, MIP-1β, RANTES, MARC, LCC-1(ref), MCP-3 and MCP-4; (4) an agent for elevating dendritic cell precursor level in the blood wherein the agonist is selected from among MIP-1α, RANTES, MARC and LCC-1(ref); (5) an agent for elevating dendritic cell precursor level in the blood wherein the agonist or a functional derivative thereof is MIP-1α or a functional derivative thereof; and (6) an agent for elevating dendritic cell precursor level in the blood wherein the functional derivative of MIP-1α is BB-10010.

Further described is: (7) an agent for elevating dendritic cell precursor level in the blood wherein the functional derivative of the agonist is an agonist to a receptor expressed in immature dendritic cells which is chemically modified with an amphipathic polymer; (8) an agent for elevating dendritic cell precursor level in the blood wherein the functional derivative of the agonist is an agonist to receptor CCR1 or CCR5 which is chemically modified with an amphipathic polymer; (9) an agent for elevating dendritic cell precursor level in the blood wherein the functional derivative of the agonist is MIP-1α, BB-10010, MIP-1β, RANTES, MARC, LCC-1(ref), MCP-3 or MCP-4 which is chemically modified with an amphipathic polymer; (10) an agent for elevating dendritic cell precursor level in the blood wherein the functional derivative of the agonist is MIP-1α, BB-10010, RANTES, MARC or LCC-1 (ref) which is chemically modified with an amphipathic polymer; (11)an agent for elevating dendritic cell precursor level in the blood wherein the functional derivative of the agonist is MIP-1α or BB-10010 which is chemically modified with an amphipathic polymer; (12) an agent for elevating dendritic cell precursor level in the blood wherein the functional derivative of the agonist is BB-10010 which is chemically modified with an amphipathic polymer; and (13) an agent for elevating dendritic cell precursor level in the blood as described in any of the above (7) to (12) wherein the amphipathic polymer is a partially alkyl-esterified styrene-maleic acid copolymer.

Moreover, further described is: (14) an agonist to a receptor expressed in immature dendritic cells which is chemically modified with an amphipathic polymer; and (15) an agonist to receptor CCR1 or CCR which is chemically modified with an amphipathic polymer.

More specifically speaking, described herein is: (16) MIP-1α, BB-10010, MIP-1β, RANTES, MARC, LCC-1(ref), MCP-3 or MCP-4 which is chemically modified with an amphipathic polymer; (17) MIP-1α chemically modified with an amphipathic polymer; (18) BB-10010 chemically modified with an amphipathic polymer; and (19) an agonist as described in any of the above (14) to (18) wherein the amphipathic polymer is a partially alkyl-esterified styrene-maleic acid copolymer or a polyethylene glycol derivative.

### Brief Description of the Drawings

Fig. 1 shows the effect of anti-MIP-1α antibody on the recruitment of dendritic cell precursors into the peripheral blood caused by administering *P*. *acnes* to B6 mice.
Fig. 2 shows changes with the passage of time in the effect of mobilizing dendritic cell precursors into the peripheral blood caused by administering MIP-1α to B6 mice.
Fig. 3 shows the effects of the proliferation of T lymphocytes in a mixed-lymphocyte reactions performed by culturing (MIP-1α)-mobilized dendritic cell precursors having been primed with antigens together with T lymphocytes.
Fig. 4 shows the effects of vaccine treatments with the use of MIP-1α-mobilized dendritic cell precursors on the proliferation of tumor cells inoculated into mice.
Fig. 5 shows the effects of MIP-1α-mobilized dendritic cell precursors on the generation of tumor-specific cytotoxic T cells *in vitro.*
Fig. 6 shows the results of an experiment for confirming the MHC class I restriction of tumor-specific cytotoxic T cells induced by MIP-1α-mobilized dendritic cell precursors.
Fig. 7 shows the effects of vaccine treatments with the use of MIP-1α-mobilized dendritic cell precursors on the metastasis of tumor cells into mouse lung.
Fig. 8 shows optical densities of individual Bu-SMA-BB10010 fractions at 280 nm.
Fig. 9 shows electrophoretic patterns of Bu-SMA-BB10010 and BB10010 employed as the starting material in Native-PAGE.
Fig. 10 shows the percentages of dendritic cells in the blood sampled 24 hours after the intravenous administration of BB10010 and Bu-SMA-BB10010 wherein shaded bars indicate the data of B220⁻CD11c⁺ cells while open bars indicate the data of B220⁺CD11c⁺.

### Best Mode for Carrying Out the Invention

As the results of studies by the present inventors with the use of MIP-1α, it is expected that ligands MIP-1α, MIP-1β, RANTES, MARC, LCC-1(ref), MCP-1, MCP-2, MCP-3, MCP-4, MCP-5, TARC, MDC, MIP-3α, MIP-3β, LARC, TECK, BLC, SDF-1, Exodus-1, Exodus-2, etc., which are ligands for receptors CCR1, CFR2, CCR4, CCR5, CCR6, CCR11, CXCR4, etc. expressed in immature dendritic cells, would have an effect of elevating dendritic cell precursor level in the blood. In particular, MIP-1α, RANTES, MARC, LCC-1(ref), etc. known as ligands commonly for CCR1 and CCR5, which seemingly relate to the migration of dendritic cells into peripheral organs as suggested by the results of the studies of the present inventors, are expected as being excellent in the effect of elevating dendritic cell precursor level in the blood.

MIP-1α which is a typical example of the active ingredient and is known as a chemokine belonging to the CC subfamily. It is a ligand (an agonist) for receptors CCR1 and CCR5. It is considered that human matured MIP-1α is composed of 70 amino acids, while it is known that MIP-1α obtained from CD8⁺T cells or the culture supernatant of HTLV-1-infected cells MT4 has 66 amino acids. In human MIP-1α, there is a nonallelic gene LD78β having different copy numbers from individual to individual and one having a sequence different in three residues from the MIP-1α of 70 amino acid type is known. They are all described in the present description.

A functional derivative of an agonist here described is a derivative of a ligand for a receptor expressed in immature dendritic cells which has an effect as an agonist. For example, a functional derivative of MIP-1α means a derivative of MIP-1α which is comparable in biological activity as an agonist to MIP-1α. As a typical example of such biological equivalents, BB-10010 (see above, European Journal of Cancer, Vol.14, No.7, pp.1023-1029 (1998)) may be cited.

It has been confirmed that when an agonist employed as an agent for elevating dendritic precursor level in the blood is chemically modified with an amphipathic polymer typified by a partially alkyl-esterified styrene-maleic acid copolymer or a polyethylene glycol, it shows an improved stability in the blood and a prolonged level-elevating effect while sustaining its activity and it topically accumulates in a cancer site due to the EPR effect. The topical accumulation of the agonist in a cancer site results in the topical accumulation of dendritic cells having been increased in the peripheral blood into the cancer site, thereby further improving the immunotherapeutic effect on cancer. Thus, it is preferred to chemically modify a functional derivative of an agonist with an amphipathic polymer. Such an agonist chemically modified with an amphipathic polymer or a biological equivalent thereof is also referred to as "a functional derivative of an agonist" in the present description.

As preferable examples of the amphipathic polymer to be used for chemically modifying a peptidic agonist or its biological equivalent, partially alkyl-esterified styrene-maleic acid copolymers may be cited. As examples of the alkyl moiety thereof, linear or branched alkyl groups having from 1 to 5 carbon atoms may be cited. These alkyl groups may be substituted by a lower alkoxy group. More specifically speaking, examples thereof include methyl, ethyl, propyl, isopropyl, n-butyl, isobutyl, s-butyl, t-butyl, n-pentyl, 3-methyl-1-butyl, 2-methyl-1-butyl, 2,3-dimethyl-1-propyl, 2-pentyl, 3-methyl-2-butyl, 3-pentyl, 2-methyl-2-butyl, methylcellosolve, ethylcellosolve and so on.

A preferable example of the partially alkyl-esterified styrene-maleic acid copolymer is a partially butyl-esterified styrene-maleic acid copolymer having an average molecular weight of form 1,000 to 10,000 and a degree of polymerization of from 1 to 100, preferably form 3 to 35, as described in Japanese Patent Publication of Examined Application 1-33119.

Other examples of the amphipathic polymer to be used for chemically modifying an agonist or its biological equivalent , polyethylene glycol (hereinafter sometimes called PEG) derivatives may be cited. The term "polyethylene glycol derivative" as used herein means a compound having a residue capable of binding to a peptide chain in an agonist or its biological equivalent in the PEG moiety represented by -O-(CH₂CH₂O)ₙ- (wherein n is an integer of from 20 to 280). Examples of the polyethylene glycol derivatives include those having a residue capable of binding to an amino group (an N-terminal amino group or an amino group in a lysine residue) in a peptide chain. Other examples of the polyethylene glycol derivatives include those having a residue capable of binding to a carboxyl group (a C-terminal carboxyl group or a carboxyl group in an aspartic acid residue or a glutamic acid residue).

Still other examples of the amphipathic polymers include polyvinylpyrrolidone and the like.

The chemically modified agonist or a biological equivalent thereof according to the present description can be obtained by chemically attaching the amphipathic polymer to the agonist or its biological equivalent optionally via a linker arm and then partially purifying. Namely, the amphipathic polymer is reacted with the agonist or its biological equivalent in a buffer solution. Then the reaction product is purified by column chromatography and fractions thus eluted are separated.

Now, this process will be described in greater detail by taking the case wherein the amphipathic polymer is a partially alkyl-esterified styrene-maleic acid copolymer for example. First, an acid anhydride of a styrene-maleic acid copolymer (SMA) obtained by radical copolymerization of styrene with maleic anhydride is dissolved in an organic solvent such as cumene. Then n-butanol is dropped into the obtained solution and reacted under stirring to thereby partially butyl-esterified the acid anhydride. Thus, a partially butyl-esterified styrene-maleic acid copolymer (Bu-SMA) can be obtained. The Bu-SMA thus obtained can be attached to an agonist or its biological equivalent by reacting them in a 0.3 M solution of sodium hydrogencarbonate (pH 8.5) so as to form an amide bond between the acid anhydride in Bu-SMA and an amino group in the agonist or its biological equivalent. The bond number of Bu-SMA to the agonist or its biological equivalent may be at least 1, preferably from 3 to 10 and still preferably from 6 to 8.

In the case of modifying an amino group in a peptide chain of an agonist or its biological equivalent by using PEG as the amphipathic polymer, it is preferable to introduce a functional group capable of reacting with an amino group (for example, a carboxyl group) into the terminus of PEG. To attach the functional group to the amino group in the peptide of the agonist or its biological equivalent, it is preferable to convert the functional group into a reactive group. To convert a carboxyl group into a reactive group, for example, it is preferable to employ the active ester method, the mixed acid anhydride method, etc.

In the active ester method, more specifically speaking, use can be made of phenyl esters such as p-nitrophenyl ester and pentafluorophenyl ester, dicarboxylic acid imide esters such as N-hydroxyphthalimide ester and N-hydroxysuccinimide ester, and hydroxyl active esters such as N-hydroxypiperidine ester. These active esters can be prepared in accordance with methods commonly employed. For example, a carboxyl group in a PEG derivative is reacted with an alcohol corresponding to such an active ester at from -20°C to room temperature for 1 to 24 hours with the use of a condensing agent such as dicyclohexylcarbodiimide or 1-ethyl-3-(3-dimethylaminopropyl)carbodiimide. Alternatively, a carboxyl group in a PEG derivative is reacted with a halide corresponding to such an active ester at from 0°C to 80°C for 1 to 72 hours in the presence of a base such as triethylamine. In the mixed acid anhydride method, a PEG derivative is reacted with isobutyl chloroformate, ethyl chloroformate, isovaleryl chloride, etc. at from -20°C to 0°C for 1 to 30 minutes in the presence of a base such as N-methylmorpholine or N-ethylpiperidine.

A PEG derivative may be directly introduced into a peptide chain of an agonist or its biological equivalent. Alternatively, it is possible to introduce the PEG derivative via a linker arm. For example, a short amino acid sequence having lysine is introduced into the target peptide chain. Then, an amino group in the lysine is modified with the PEG derivative. In the modification reaction, it is preferred to employ the PEG derivative in an amount from 10 to 30 times by mol as much as the amino group number contained in the peptide chain to be modified.

In the case of modifying a carboxyl group in a peptide chain of an agonist or its biological equivalent with the use of PEG as the amphipathic polymer, it is preferable to introduce a functional group capable of reacting with the carboxyl group (for example, an amino group) into the terminus of PEG.

It is expected that the thus obtained peptidic agonist or its biological equivalent chemically modified with the amphipathic polymer would accumulate selectively in a target diseased site such as solid tumor due to the so-called EPR effect.

In the agent according to the present description for elevating dendritic cell precursor level in the blood, it is preferable to parenterally administer the agonist or its biological equivalent (i.e., a protein) employed as the active ingredient. For example, it is preferable to administer the agent as an injection or the like in the form of an aseptic solution or suspension in water or a pharmaceutically acceptable liquid. An aseptic composition for injection can be prepared in accordance with formulation means commonly employed, for example, dissolving or suspending the active ingredient in a vehicle such as injection water or a natural vegetable oil. As an aqueous liquid for injection, use can be made of physiological saline, an isotonic solution containing glucose and other auxiliaries (for example, D-sorbitol, D-mannitol or sodium chloride). It is also possible to further employ an appropriate dissolution aid such as an alcohol (for example, ethanol), a polyalcohol (for example, propylene glycol or polyethylene glycol), a nonionic surfactant (for example, Polysorbate 80™ or HCO-50) and so on. As an oily liquid, use can be made of, for example, sesame oil, palm oil or soybean oil. As a dissolution aids, it is also possible to further blend benzyl benzoate, benzyl alcohol, etc. Moreover, use may be made of a buffer agent (for example, a phosphate buffer solution or a sodium acetate buffer solution), a soothing agent (for example, benzalkonium chloride or procaine hydrochloride), a stabilizer (for example, human serum albumin or polyethylene glycol), a preserving agent (for example, benzyl alcohol or phenol), an antioxidant and so on.

The agonist or its biological equivalent having Bu-SMA attached thereto which is obtained can be used as a water-soluble or oily injection. A water-soluble injection is employed mainly in intravenous administration. An oily injection, which is prepared by uniformly dispersing the agonist or its biological equivalent having Bu-SMA attached thereto in an oily agent such as Lipiodol, is administered to a target diseased site such as solid tumor via, for example, a catheter fixed in the upstream of the tumor feeding artery. The present inventors have found out that the agonist or its biological equivalent having Bu-SMA attached thereto binds to albumin in the blood and thus behaves as a high-molecular weight compound and that the Bu-SMA-attached compound is soluble in an oily agent. Accordingly, it is expected that, when topically injected into the desired artery, the Bu-SMA-attached agonist or its biological equivalent in the form of an oily preparation would accumulate selectively in the target diseased site such as solid tumor due to the so-called EPR effect.

The preparation thus obtained can be administered to humans or other mammals. Although the dose of the active ingredient according to the present description varies depending on the disease to be treated, the subject of administration, the administration route and so on, the single dose of the active ingredient in parenteral administration to an adult (60 kg in body weight) ranges from, for example, about 0.01 to 10 mg, preferably from about 0.1 to 5 mg. To other animals, it may be administered in a dose calculated in terms of body weight with the use of 60 kg as the basis.

### Referential Example 1

### Recruitment of dendritic cell precursor into the blood by P. acnes and inhibition of the recruitment by anti-MIP-1α antibody

*P. acnes* (1 mg/animal; American Type Culture Collection 118289) was injected via the tail vein into B6 mice (female, aged 8 to 10 weeks; purchased from CLEA Japan, Inc.) to recruit dendritic cell precursors into the blood. Six hours before the *P. acnes* administration, anti-MIP-1α antibody (a goat polyclonal antibody; 100 µg/µl; manufactured by Genzyme/Techne) having an inhibitory activity was injected via the tail vein to some of these mice. As a negative control, goat IgG (manufactured by Sigma-Aldrich) was similarly administered.

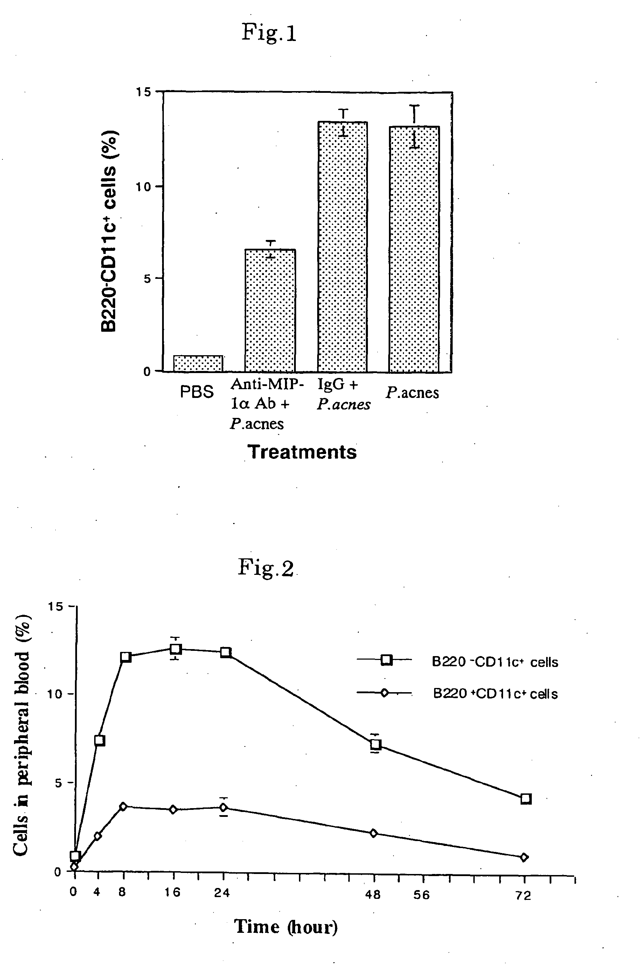

Three days after the *P. acnes* administration, blood was collected from each animal with the use of heparin and then separated by using NycoPrep (manufactured by Axis-Shield) to obtain peripheral blood mononuclear cells. Next, these peripheral blood mononuclear cells were reacted with FITC-labeled anti-CD11c antibody (Clone HL3; manufactured by PharMingen) and PE (phycoerythrin)-labeled anti-B220 antibody (Clone RA3-6B2; manufactured by PharMingen) at 4°C and then analyzed with a flow cytometer (EPICS-Elite; manufactured by Coulter). Thus, the percentages of dendritic cell precursors in the peripheral blood mononuclear cells were calculated by referring BB220 antigen-negative and CD11c antigen-positive cells as the dendritic cell precursors. Fig. 1 shows the results wherein "PBS" stands for the result of a group to which PBS alone was administered as a control.

The administration of *P. acnes* resulted in a drastic increase in the percentage of the dendritic cell precursors in the peripheral blood mononuclear cells compared with the non-administration group. This fact suggests that dendritic cell precursors were recruited into the blood. It was also confirmed that the increase in the appearance frequency of the dendritic cell precursors in the peripheral blood mononuclear cells caused by the administration of *P. acnes* was almost halved in the group to which anti-MIP-1α antibody was administered.

### Referential Example 2

### Method of producing MIP-1α

Human MIP-1α gene was obtained by the PCR method. Then this human MIP-1α gene was integrated into an expression shuttle vector pNCMO2 and amplified in *Escherichia coli.* Then the human MIP-1α gene expression vector was transferred into *Brevibacillus chonshinensis (B. chonshinensis)* HPD31S5. This *B. chonshinensis* having the human MIP-1α gene transferred thereinto was cultured and the supernatant was collected. To this culture supernatant was added ammonium sulfate to achieve 40% saturation. After forming a precipitate, the supernatant was separated by centrifuging and ammonium sulfate was further added thereto to give 60% saturation. After forming a precipitate, the precipitate was collected by centrifuging. Then this precipitate was dissolved in a tris hydrochloride buffer (pH 8.0) and the obtained solution was fractionated by anion exchange chromatography (Q Sepharose; manufactured by Amersham). From the obtained fractions, those containing MIP-1α were combined and lysed by adding ammonium sulfate (final concentration: 1.5 M). The obtained lysate was fractionated by hydrophobic chromatography (RESOURCE PHE; manufactured by Amersham). From the obtained fractions, those containing MIP-1α (unadsorbed fractions) were combined and subjected to ammonium sulfate precipitation by adding ammonium sulfate (final concentration: 60% saturation). The precipitate was dissolved in a tris hydrochloride buffer (pH 8.0). The solution was fractionated again by anion exchange chromatography (RESOURCE Q: manufactured by Amersham). From the obtained fractions, those containing MIP-1α were combined and dialyzed against 20 mM NH₄HCO₃ (pH 8.5) to thereby remove the tris salt. By centrifuging the precipitate obtained by this treatment, purified human MIP-1α was obtained. After freeze-drying, the product was dissolved in PBS and used in the following experiments.

### Referential Example 3

### Method of producing BB10010 (expression and purification)

cDNA of BB10010 was prepared by site-specific mutation with Quik Change Kit (manufactured by Stratagene) with the use of human MIP-1α cDNA as a template. Namely, 1 µl of Pfu-turbo (2.5 U/µl) was added to 50 µl of a reaction system containing 125 ng of mutation primers RQ1:5'-CCAGCGAAGCCGGCAGGTCTGTGCTGACCCAG-3' (SEQ ID NO:1) and RQ2:5'-CTGGGGTCAGCACAGACCTGCCGGCTTCGCTTGG-3' (SEQ ID NO:2), 10 ng of the template plasmid DNA and 50 µM of dNTP. After denaturation at 95°C for 30 seconds, the reaction was performed for 12 cycles with each cycle consisting of 30 seconds at 95°C, 1 minute at 55°C and 7 minutes at 68°C. Subsequently, 1 µl of a restriction enzyme DpnI was added to the reaction system and the reaction was carried out at 37°C for 1 hour. Thus, the template DNA was cut off and the mutated plasmid alone was recovered. After confirming that the mutation site had been correctly substituted by DNA sequence analysis, PCR was carried out by a conventional method with the use of MIPM2 primer 5'-CATGCCATGGCTTTCGCTTC ACTTGCTGCTGACAC (SEQ ID NO:3) and MIPRV primer 5' -CGCGGATCCTCAGGCATCAGCTCTAG (SEQ ID NO:4). After cutting with restriction enzymes Nco1 and BamH1, the obtained fragment was inserted into the restriction enzyme site of a *Bacillus brevis* expression vector pNCM02.

Next, *Brevibacillus chonshinensis* HPD31 strain was transformed thereby and cultured in a TMN medium contained in a 20 L jar fermenter at 30°C for 30 days to thereby allow secretion and expression. The supernatant of the culture medium was subjected to sterile filtration through a 0.45 um filter to give a starting material for purification. To this medium supernatant, a phosphate buffer (0.1 M Na phosphate) at a 10-fold concentration was added to adjust the final pH value to 7.0. Then the liquid mixture was supplied at 5 ml/min into a heparin column (5 ml in size; Amersham Bioscience) having been equilibrated with a 10 mM phosphate buffer (pH 7.0) with the use of an AKTA-prime system (Amersham Bioscience) to thereby adsorb BB10010. Then, the column was eluted under gradient with a phosphate buffer (pH 6.5) containing 1 M sodium chloride. The presence of BB10010 in fractions was confirmed by SDS polyacrylamide gel electrophoresis. The peak fraction was dialyzed against a 50 mM formate buffer solution (pH 4.0) . Next, the eluate was supplied into a cation exchange chromato SP-FF (1ml in size, Amersham Bioscience) at a speed of 1 ml/min to adsorb BB10010. Next, the column was eluted under gradient with a formate buffer (pH 3.8) containing 1 M sodium chloride. The presence of BB10010 in fractions was confirmed by SDS polyacrylamide gel electrophoresis. The peak fraction was subjected to gel filtration chromatography (packing agent: Bio-Gel P60, manufactured by Bio-Rad Laboratories, mobile phase: 20 mM ammonium hydrogencarbonate solution (pH 8.5), column size: 1.6x83 cm) to thereby purify BB10010. Then, the peak fraction was freeze-dried.

### Example 1

### Activity of MIP-1α of mobilizing dendritic cell precursor into mouse blood

Purified MIP-1α (5 µg/animal) obtained by the production method of Referential Example 2 was injected via the tail vein into B6 mice (female, aged 8 to 10 weeks; CLEA Japan, Inc.) to mobilize dendritic cell precursors into the blood. 4, 8, 16, 24, 48 and 72 hours after the administration of MIP-1α, the blood was collected from the mice with the use of heparin and then analyzed with a flow cytometer as in Referential Example 1 to thereby calculate percentages of dendritic cell precursors in peripheral blood mononuclear cells.

Fig. 2 shows the results. As the line plot with open squares (□) in Fig. 2 shows, the administration of MIP-1α resulted in a drastic increase in the percentage of dendritic cell precursors (B220⁻CD11c⁺ cells) in peripheral blood mononuclear cell within a short period of time. Eight hours after the administration, the percentage attained a peak (12.45%±0.49) which was sustained until 24 hours after the administration. Since the dendritic cell precursor concentration before the MIP-1α administration (at the point of time zero in Fig. 2) was from 0.5 to 1%, it can be understood that the dendritic cell precursor level in the blood was increased 12- to 24-fold.

As the line plot with open rhomboids (◇) in Fig. 2 shows, the administration of MIP-1α resulted in an increase in the percentage of dendritic cell precursors (B220⁺CD11c⁺ cells) in peripheral blood mononuclear cell within a short period of time. Eight hours after the administration, the percentage attained a peak which was sustained until 24 hours after the administration. According to recent studies by the present inventors, it has been confirmed that B220⁺CD11c⁺ cells which are the plasma cell-derived dendritic cell precursors directly bind to high endothelial venules in inflammatory lymph nodes depending on E selectin and CXCL9 and migrate, thereby supporting the function of B220⁻CD11c⁺ cells which are bone marrow-derived dendritic cells. Also, it has been clarified by the present inventors that B220⁺CD11c⁺ cells produce interferon γ and promote the activation of killer cells. Based on these findings, it is considered that the increase in the B220⁺CD11c⁺ cell level in the peripheral blood is highly meaningful in immunotherapy.

### Example 2

### Analysis of function of dendritic cell precursor mobilized into mouse blood by MIP-1α

The following experiments were carried out to analyze whether or not the dendritic cell precursors mobilized into mouse blood by MIP-1α have the same properties as the dendritic cell precursors recruited by *P. acnes.*
<1> Preparation of peripheral blood-derived dendritic cell precursor
   Dead *P*. *acnes* cells (1 mg/animal) or purified MIP-1α (5 µg/animal) obtained by the production method of Referential Example 2 was injected via the tail vein into B6 mice (female, aged 8 to 10 weeks; purchased from CLEA Japan, Inc.). Three days after the administration of dead *P. acnes* cells or 16 hours after the administration of MIP-1α, the blood was collected by using heparin from heart of each mouse under anesthesia (0.8 ml/animal) . The thus collected blood was separated by using NycoPrep to obtain peripheral blood mononuclear cells. Next, these peripheral blood mononuclear cells were reacted with FITC-labeled anti-CD11c antibody and PE-labeled anti-B220 antibody at 4°C for 30 minutes. Then B220 antigen-negative and CD11c antigen-positive cells were separated with a cell sorter (EPICS-Elite; manufactured by Coulter). The cells thus separated were cultured in the presence of GM-CSF (4 ng/ml; manufactured by Kirin Brewery) and IL-4 (10 ng/ml; manufactured by Genzyme/Thech) for 5 days to give peripheral blood-derived dendritic cell precursors.
<2> Preparation of bone marrow-derived dendritic cell precursor
   Bone marrow cells were collected from B6 mouse thighbone and tibia. Then these bone marrow cells were separated by using NycoPrep to obtain bone marrow mononuclear cells. These bone marrow mononuclear cells were cultured in a Petri dish for 10 to 12 hours and unbound bone marrow mononuclear cells were collected. The unbound bone marrow mononuclear cells were mixed with magnetic bead-labeled c-kit antibody and cultured. Next, c-kit-positive cells were separated with the use of a magnetic cell sorter (MACS; manufactured by Miltenyi Biotec). These c-kit-positive cells were cultured at a concentration of 2.5x10⁵ cells/ml in the presence of SCF (provided by Dr. Sudo, Toray), Flt3L (manufactured by Immunex), GM-CSF (4 ng/ml) and IL-4 (10 ng/ml) for 7 to 9 days to give bone marrow-derived dendritic cell precursors.
<3> Priming dendritic cell precursor with antigen
   A cancer cell line lysate employed as an antigen was prepared by repeatedly freezing and thawing cancer cells thrice. The peripheral blood-derived dendritic cell precursors (derived from the mice administered with dead *P*. *acnes* cells or purified MIP-1α) and the bone-marrow-derived dendritic cell precursors were mixed with the cancer cell lysate to give a ratio of dendritic cell precursors:cancer cells of 1:3 followed by culturing for 20 hours. Then the dendritic cell precursors thus primed with the cancer cell line lysate were collected, treated with Mitomycin C (10 µg/ml) and washed twice to give antigen-primed dendritic cell precursors.
<4> T lymphocyte proliferation measurement using antigen-primed dendritic cell precursor
   CD3-positive T lymphocytes were prepared from B6 mouse spleen cells by using a magnetic cell sorter (MACS; manufactured by Miltenyi Biotec). Then these T lymphocytes were mixed with the antigen-primed dendritic cell precursors (the bone marrow-derived dendritic cell precursors having been primed with the B16 cancer cell line lysate) to give a ratio of 1:20 and cultured in a 24-well plate in the presence of IL-2 and IL-7. After culturing the T lymphocytes for 5 days, the culture was continued while replacing 50% of the medium with a fresh one at intervals of 2 or 3 days. On the days 7 and 14, antigen-primed dendritic cell precursors were newly added to thereby re-prime the T lymphocyte under culturing. On the day 21 of the culture, the T lymphocytes were collected. Subsequently, the T lymphocytes having been cultured were mixed with the peripheral blood-derived dendritic cell precursors (derived from the mice administered with dead *P*. *acnes* cells or purified MIP-1α) having been primed with the B16 cancer cell line lysate, the bone-marrow-derived dendritic cell precursors having been primed with the MMC-treated B16 cancer cell line lysate, the dendritic cell precursors having been primed with an EL4 cancer cell line lysate, unprimed dendritic cell precursors or the cancer cell line lysate alone in a 96-well round bottomed plate (manufactured by Nunc) and cultured at 37°C for 4 to 5 days. After the completion of the culture, 15 µl/well of a 5 mg/ml MMT (3-(4, 5-dimethylthiazolyl -2-yl)-2,5-diphenyltetrazolium bromide) was added and the culture was continued at 37°C for additional 4 hours. After the completion of the culture, the absorbance of the contents in each well was measured at 550 nm, thereby measuring the proliferation of the T lymphocyte.

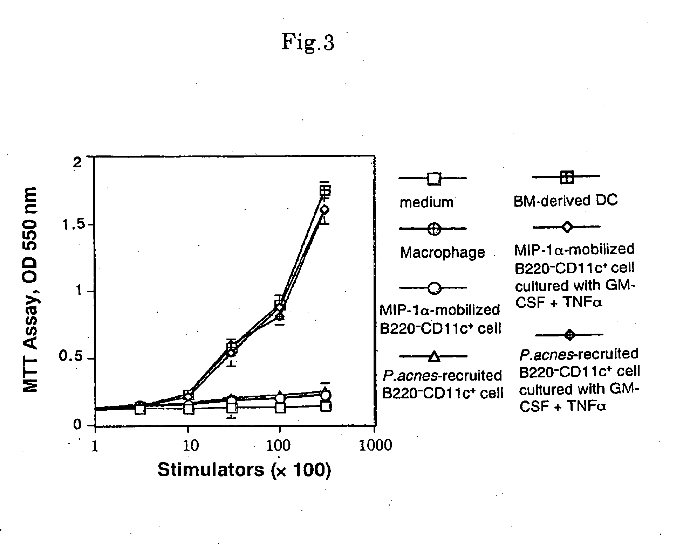

   Fig. 3 shows the results. The peripheral blood-derived dendritic cells obtained from the purified MIP-1α-administered mouse, which had been primed with the B16 cancer cell line lysate, potentiated the proliferation of the cultured T lymphocytes similar to the peripheral blood-derived dendritic cell precursors obtained from the dead *P. acnes* cell-administered mouse or the bone marrow-derived dendritic cell precursors. In contrast, no potentiating activity on the proliferation of the cultured T lymphocytes was observed in the case using the dendritic cell precursors having been primed with the EL4 cancer cell line lysate, the unprimed dendritic cell precursors or the cancer cell lysate alone.
<5> Discussion on the effect of immunization on mouse tumor proliferation
   B6 mice were divided into groups (each having 8 animals) and subcutaneously injected in the abdominal side with the peripheral blood-derived dendritic cell precursors (obtained from the mice administered with dead *P. acnes* cells or purified MIP-1α) having been primed with the B16 cancer cell line lysate, the bone marrow-derived dendritic cell precursors having been primed with the B16 cancer cell line lysate, the dendritic cell precursors having been primed with the EL4 cancer cell line lysate, unprimed dendritic cell precursors (1x10⁶/animal in each case), the cancer cell line lysate alone or PBS. After 14 days, B16 cancer cells (2x10⁵/animal) were subcutaneously inoculated into abdominal side of the mice. Following the inoculation, the tumor area was measured at intervals of 3 days to judge the proliferation of the tumor. Simultaneously, surviving mice were counted.

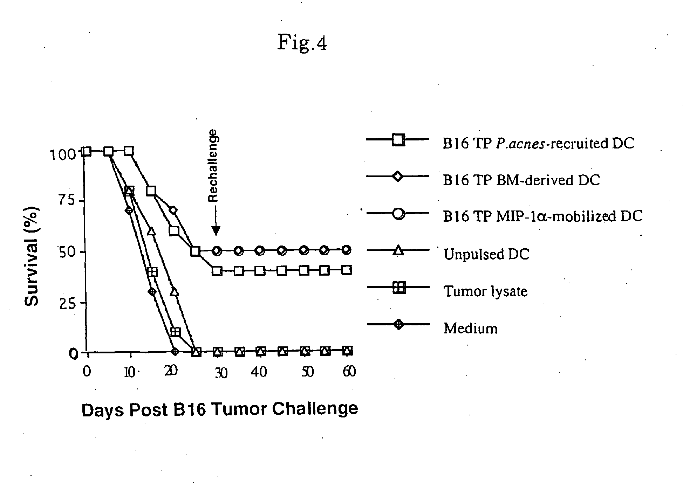

   Fig. 4 shows the results. By immunizing the mice with the peripheral blood-derived dendritic cell precursors obtained from the mice administered with MIP-1α which had been primed with the B16 cancer cell line lysate, the proliferation of the subcutaneously inoculated B16 cancer cells was remarkably inhibited. Moreover, the tumor disappeared in 50% (5/10) of mice and the survival was prolonged by 60 days or longer. Similar effects were observed in the peripheral blood-derived dendritic cell precursors obtained from the mice administered with dead *P. acnes* cells and the bone marrow-derived dendritic cell precursors. In contrast thereto, all animals died within 20 days after the cancer inoculation in the group of the dendritic cell precursors primed with the EL4 cancer cell line lysate and the group of the cancer cell line lysate alone.
<6> Measurement of tumor-specific cytotoxic activity
   By the same method as in the above <4>, CD3-positive T lymphocytes were prepared from B6 mouse spleen cells and cultured together with the peripheral blood-derived dendritic cell precursors (obtained from the mice administered with dead *P. acnes* cells or purified MIP-1α) having been primed with the B16 cancer cell line lysate, the bone marrow-derived dendritic cell precursors having been primed with the B16 cancer cell line lysate or the cancer cell line lysate alone to thereby give cultured T lymphocytes. The cultured T lymphocytes were serially diluted and added to a 96-well plate at a ratio of 100 µl/well. Next, a cell suspension of the B16 cancer cell line was added at a ratio of 100 µl/well. After culturing at 37°C for 10 hours, the plate was centrifuged and 100 µl/well of the supernatant was transferred into a fresh 96-well plate. Then a reaction solution in a cytotoxicity detection kit (LDH; manufactured by Boehringer Mannheim) was added to each well at a ratio of 100 µl/well. After reacting the reaction plate at room temperature for 30 minutes, the absorbance thereof was measured at 490 nm with a plate absorption meter.

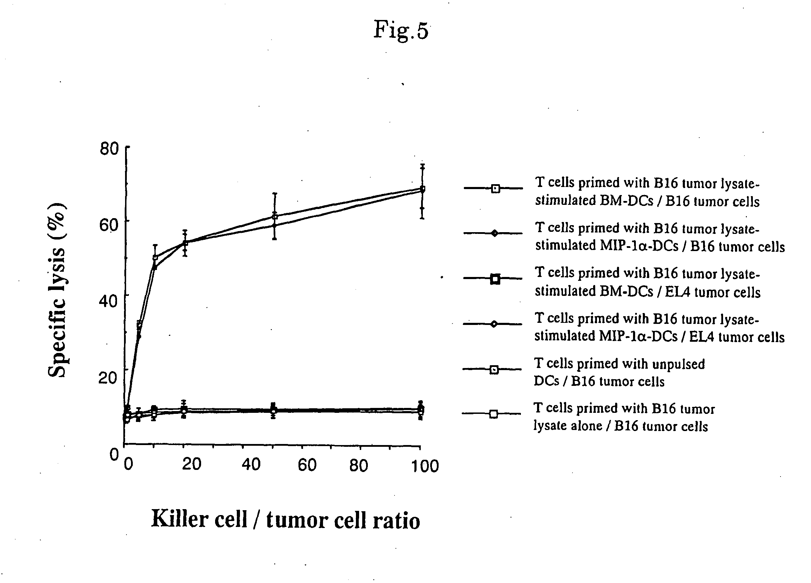

   Fig. 5 shows the results. The T lymphocytes cultured together with the peripheral blood-derived dendritic cell precursors obtained from the mice administered with purified MIP-1α and having been primed the B16 cancer cell line lysate efficiently injured B16 cancer cells but not EL4 cancer cells. Similarly, the T lymphocytes cultured together with the peripheral blood-derived dendritic cell precursors obtained from the mice administered with dead *P. acnes* cells and having been primed the B16 cancer cell line lysate or the bone marrow-derived dendritic cell precursors efficiently injured B16 cancer cells. In contrast, the T lymphocytes cultured together with the cancer cell line lysate alone did not injure B16 cancer cells.
<7> Spleen cells were collected from B6 mice showing disappearance of cancer cells on the day 60 after the B16 cancer cell line inoculation from among the B6 mice immunized with the peripheral blood-derived dendritic cell precursors (obtained from the mice administered with MIP-1α) having been primed the B16 cancer cell line lysate and the B6 mice immunized with the bone marrow-derived dendritic cell precursors in the above <5>. CD8-positive T lymphocytes (1x10⁶) were separated from the spleen cells and cultured together with MMC-treated B16 cancer cell line (1x10⁵) for 5 days. Then the cultured T lymphocytes were serially diluted and added to a 96-well plate at a ratio of 100 µl/well. Next, a cell suspension of the B16 cancer cell line was added at a ratio of 100 µl/well. After culturing at 37°C for 10 hours, the plate was centrifuged and 100 µl/well of the supernatant was transferred into a fresh 96-well plate. Then a reaction solution in a cytotoxicity detection kit (LDH; manufactured by Boehringer Mannheim) was added to each well at a ratio of 100 µl/well. After reacting the reaction plate at room temperature for 30 minutes, the absorbance thereof was measured at 490 nm with a plate absorption meter to thereby assay the cytotoxic activity. In an experiment on MHC class I restriction inhibition, the B16 cancer cells were treated with anti-MHC class I antibody (anti-H2Kb/H2Db antibody) or a control antibody (anti-H2Ds antibody) at 37°C for 30 minutes before the addition.

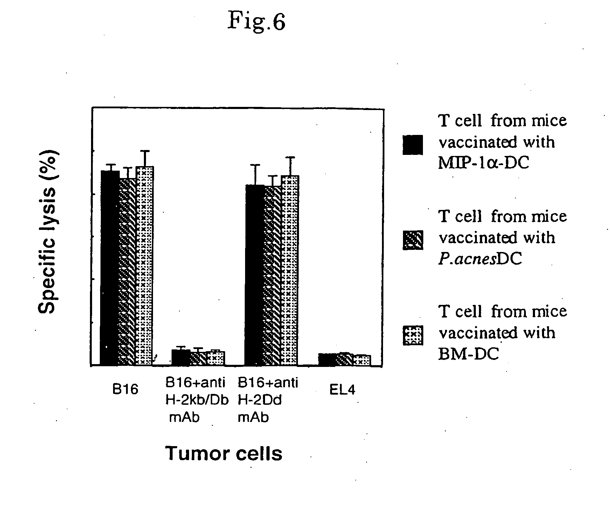

   Fig. 6 shows the results. The cultured T lymphocytes efficiently injured B16 cancer cells but not EL4 cancer cells. Although this activity was completely inhibited by treating the T lymphocytes with the anti-MHC class I antibody, the control antibody did not exert such an effect.
<8> On the days 0 and 7, B6 mice were subcutaneously immunized in the abdominal side with the peripheral blood-derived dendritic cell precursors (obtained from the mice administered with dead *P*. *acnes* cells or purified MIP-1α) having been primed with the B16 cancer cell line lysate, the bone marrow-derived dendritic cell precursors having been primed with the B16 cancer cell line lysate, the dendritic cell precursors having been primed with an EL4 cancer cell line lysate, unprimed dendritic cell precursors (1x10⁶/animal in each case), the cancer cell line lysate alone or PBS. Seven days after the second immunization, spleen cells were collected from the immunized mice. Then, CD4-positive cells and CD8-positive cells, i.e. T cells, were separated from these spleen cells by using a cell sorter. These T cells were mixed with MMC-treated B16 cancer cells and cultured by using a 24-well plate for 48 hours. Next, the cytotoxic activity was assayed with the use of the cells thus cultured. Further, the IFNγ concentration in the supernatant of the cultured cells was measured by using an IFNγ ELISA system (manufactured by Endogen).
<9> Confirmation of effect metastasis into lung
   B16 cancer cells (1x10⁶/animal) were injected into B6 mice via the tail vein. On the days 3 and 7 following the injection of the cancer cells, the peripheral blood-derived dendritic cell precursors (obtained from the mice administered with MIP-1α) having been primed with the B16 cancer cell line lysate, the bone marrow-derived dendritic cell precursors having been primed with the B16 cancer cell line lysate, unprimed dendritic cell precursors (1x10⁶/animal in each case), the cancer cell line lysate alone or PBS were each injected into the mice via the tail vein. On the day 21 following the cancer cell injection, mouse lungs were surgically taken out and metastatic foci were counted, thereby judging the degree of metastasis.

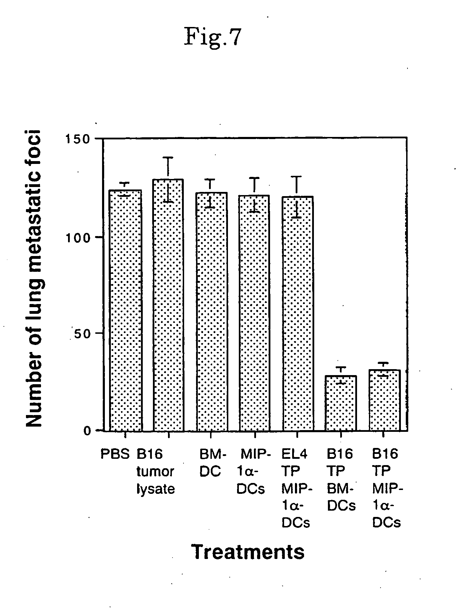

   Fig. 7 shows the results wherein the data are expressed in the means of individual groups each having 3 animals. In the mice inoculated with the cancer cells and then immunized with the peripheral blood-derived dendritic cell precursors (obtained from the mice administered with MIP-1α) and the bone marrow-derived dendritic cell precursors having been primed with the B16 cancer cell line lysate, metastasis of the B16 cancer cells into lungs was drastically inhibited. However, the T lymphocytes cultured together also injured the B16 cancer cells. In contrast, no such metastasis inhibitory effect was observed in the mice immunized with the unprimed dendritic cell precursors.

### Example 3

### Method of producing Bu-SMA-attached BB10010

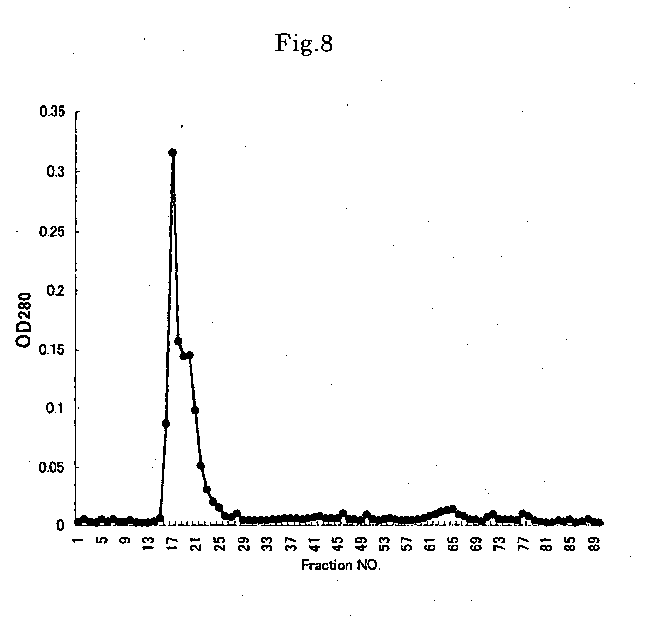

BB10010 obtained in Referential Example 3 was dissolved in a 0.8M sodium hydrogencarbonate buffer (pH 8.5) to give a concentration of 2 mg/ml. To 1 ml of this solution, 2.6 mg of Bu-SMA dissolved in diethylformamide (molar ratio Bu-SMA:BB10010=10:1) was slowly added and the resulting mixture was reacted at 27°C overnight. After the completion of the reaction, the reaction mixture was subjected to gel permeation chromatography (packing agent: Bio-Gel P60, manufactured by Bio-Rad Laboratories, mobile phase: 20 mM ammonium hydrogencarbonate solution (pH 8.5), column size: 1.6x83 cm) to thereby purify Bu-SMA-attached BB10010. The eluate was collected into 90 test tubes in 2 ml portions (Fig. 8). Based on the absorbances at 280 nm, the fractions eluted in test tubes No.16 to No.18 (6 ml in total) were then freeze-dried to give Bu-SMA-attached BB10010 (hereinafter expressed as "Bu-SMA-BB10010") as a white powder.

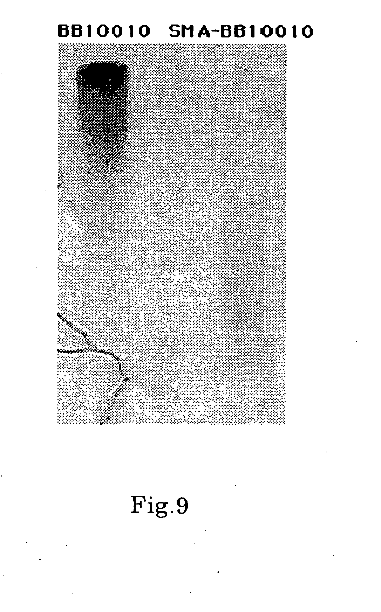

Fig. 9 shows the results in Native-PAGE (undenatured 10-20% gradient polyacrylamide gel electrophoresis) of Bu-SMA-BB10010 thus obtained and BB10010 employed as the starting material. These two substances show obviously different migration distances. This is seemingly because Bu-SMA-BB10010 was more easily electrophoresed in the anode side, since an amino group in lysine of BB10010 was modified by SMA and the positive surface charge was thus lowered. Based on the difference in migration distance between these substances and quantification results obtained by reacting Bu-SMA-BB10010 in the fractions No.16 to No.18, unreacted Bu-SMA and amino groups in BB10010 with a fluorescent reagent Fluorescamine (0.3 mg/ml in acetone), measuring the fluorescences with a spectrofluoromether at an excitation wavelength of 390 nm and a fluorescence wavelength of 475 nm, it was estimated that 2 to 3 SMA units were attached per molecule.

### Example 4

80 ml of the blood was collected from a normal human subject and centrifuged at 1600 rpm together with Polymorphprep (Daiichikagaku) at 20°C for 35 minutes to thereby separate the mononuclear cell layer. To remove lymphocytes and neutrophils, use was made of the plastic adsorption method. Namely, the mononuclear cell layer was suspended in a culture dish, which had been coated with RPMI1640 (SIGMA) containing 10% FCS at 37°C for 30 minutes, and the concentration was adjusted to 2x10⁵ cells/cm² with RPMI1640 containing 10% FCS. Then, the plate was allowed to stand at 37°C under CO₂ partial pressure for 1 hour and thus mononuclear cells were adsorbed by the dish. After removing the liquid culture medium, the dish was washed twice with RMPM1640 containing 1% FCS which had been preliminarily heated to 37°C. Next, RPMI1640 containing 1% FCS at 4°C was added and the mixture was allowed to stand on ice for 30 minutes. Subsequently, mononuclear cells were collected by pipetting and the mononuclear cell suspension was centrifuged at 1600rpm at 4°C. After removing the supernatant, the concentration was adjusted to 1x10⁶ cells/ml with RPMI1640 containing 1% FCS. A 50 µl portion of this suspension was placed in the upper part of a 46-well chemotaxis chamber (IEDA TRADING) while a chemotactic factor was placed in the lower part. Then, the chamber was allowed to stand under CO₂ partial pressure for 90 minutes. The membrane filter employed as the boundary had a pore size of 5 µm. Then the cells were stained successively with the solution I of Diff Quick (INTERNATIONAL REAGENT) for 10 seconds and the solution II for 10 seconds and stained cells were counted under a microscope. As a result, a fraction containing Bu-SMA-BB10010 showed a mononuclear cell chemotaxis activity depending on concentration and this activity was higher by twice or more than the unmodified BB10010 even at a concentration of about 1/100 level. It was thus confirmed that BB10010 chemically modified with Bu-SMA still sustains its biological activity and this activity exceeds the unmodified one.

### Example 5

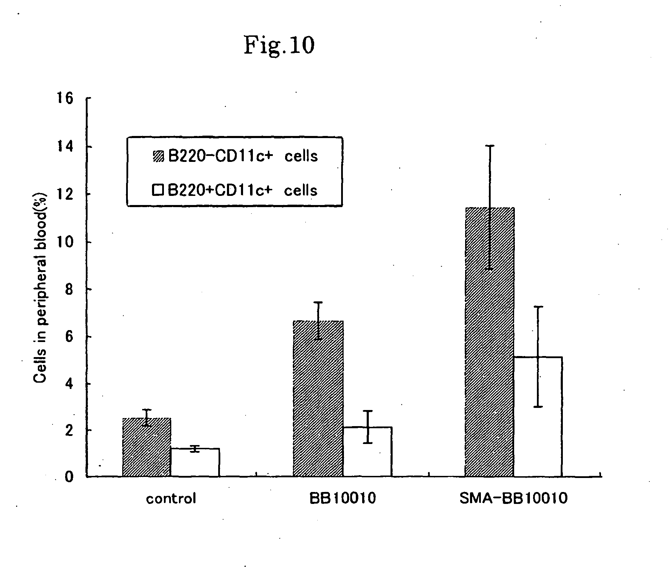

To compare Bu-SMA-BB10010 with BB10010 in the function of mobilizing dendritic cells into the blood, the following experiment was carried out. 200 µg/200 µl of Bu-SMA-BB10010 (fractions No.16 to NO.18) or 200 µg/200 µl of unmodified BB10010 was injected into BALB/c mice (aged 9 weeks, male; Seac Yoshitomi) via the tail vein. After 24 hours, venous blood was collected from the mesenteric vein. After adjusting the concentration to 5x10⁵ cells/50 µl with PBS (-) +2%FCS, the thus obtained mononuclear cells were reacted on ice in the dark with FITC-labeled anti-CD11c antibody (FITC CONJUGATED HAMSTER ANTIMOUSE CD11c; Pharmingen) and PRE-labeled anti-B220 antibody (R-Pe CONJUFATED RAT ANTIMOUSE 45R/B220; Pharmingen) diluted 25-fold and 200-fold respectively. Next, the ratio of cells having FACS (manufactured by BECTON DICKINSON) fluorescent-labeled surface antigen was determined and quantified (Fig. 10).

As a result, the percentage of CD11c(+) B220 (-) cells in the mononuclear cells was elevated to 11.45±2.59% in the Bu-SMA-BB10010 administration group, namely, 4.5 times higher than the control group (2.25±0.34%) and 1.7 times higher than the unmodified BB10010 administration group (6.65±0.78%). Namely, it was confirmed that the modification with SMA caused an elevated stability in the blood and a potentiated function of mobilizing dendritic cells into the blood. Similarly, the percentage of CD11c (+) B220 (+) cells was elevated to 5.14±2.12% in the Bu-SMA-BB10010 administration group, namely, 4.3 times higher than the control group (1.19±0.13%) and 2.4 times higher than the unmodified BB10010 administration group (2.12±0.69%). Namely, a potentiated function of mobilizing these cells into the blood was observed too.

### Industrial Applicability

It is reported that Kupffer cells produce MIP-1α in the liver and DC precursor cells express a receptor CCR5 corresponding thereto (J. Exp. Med., 198:35-49 (2001)). Based on these facts, it is assumed that once inflammation occurs, dendritic cell precursors would rush to search for an antigen at a speed comparable to neutrophils regardless of a need for an immune response and, in the case where there is an antigen, the dendritic cell precursors induce an immune response and lessen risk *in vivo (*Igaku no Ayumi, Vol.200, No. 6, pp.472-476 (2002)).

It is also reported that CD34⁺ cells which are stem cells capable of differentiating into dendritic cells are contained in the peripheral blood and mobilized by MIP-1α. Moreover, it is known that MIP-1α is usable in culturing and proliferating CD34⁺ cells *in vitro* (USP 5922597 (1999)).

However, it has never been known so far that exogenously administered MIP-1α contributes directly to the mobilization of dendritic cell precursors into the blood and the concentration of dendritic cell precursors in the blood is elevated several ten-fold thereby. Namely, these facts have been found out for the first time by the present inventors.

As described in detail in Example 2, moreover, dendritic cell precursors the level of which in the blood is elevated by MIP-1α exhibit a satisfactory effect as a vaccine *in vivo.*

By chemically modifying a biological equivalent of MIP-1α with an amphipathic polymer, the effect of mobilizing dendritic cell precursors is remarkably enhanced. Based on this fact, it is expected that similar effects might be established in other agonists and biological equivalents thereof.

Accordingly, it can be concluded that the present description makes it possible to collect a sufficient amount of dendritic cell precursors from a patient, thereby opening the way to the practical use of immunotherapy with the use of dendritic cells.

## Claims

1. A pharmaceutical composition comprising BB-10010 which is chemically modified with a partially butyl-esterified styrene-maleic copolymer as the active ingredient.

2. Use of BB-10010, which is chemically modified with a partially butyl-esterified styrene-maleic copolymer, for the preparation of a pharmaceutical composition for treating a solid tumor.

3. BB-10010, which is chemically modified with a partially butyl-esterified styrene-maleic copolymer.

## Patentansprüche

1. Arzneimittel, das als Wirkstoff BB-10010 umfasst, das mit einem partiell mit Butyl veresterten Styrol-Maleinsäure-Copolymer chemisch modifiziert ist.

2. Verwendung von BB-10010, das mit einem partiell mit Butyl veresterten Styrol-Maleinsäure-Copolymer chemisch modifiziert ist, für die Herstellung eines Arzneimittels zur Behandlung eines soliden Tumors.

3. BB-10010, das mit einem partiell mit Butyl veresterten Styrol-Maleinsäure-Copolymer chemisch modifiziert ist.

## Revendications

1. Composition pharmaceutique comprenant de la BB-10010, qui est chimiquement modifiée avec un copolymère styrène-maléique partiellement butyl-estérifié, en tant qu'agent actif.

2. Utilisation de la BB-10010, qui est chimiquement modifiée avec un copolymère styrène-maléique partiellement butyl-estérifié, pour la préparation d'une composition pharmaceutique pour le traitement d'une tumeur solide.

3. BB-10010, qui est chimiquement modifiée avec un copolymère styrène-maléique partiellement butyl-estérifié.


### SEQUENCE LISTING

<110> Effector Cell Institute
   MATSUSHIMA, Kouji
<120> Increasing of Dendritic Cell precursors in peripheral blood
<130> NTK03-1564W0
<140>
   <141>
<150> JP 2002-310090
   <151> 2002-10-24
<150> JP 2003-170091
   <151> 2003-06-13
<160> 4
<170> PatentIn version 3.1
<210> 1
   <211> 32
   <212> DNA
   <213> Homo sapiens
<400> 1
   ccagcgaagc cggcaggtct gtgctgaccc ag 32
<210> 2
   <211> 34
   <212> DNA
   <213> Homo sapiens
<400> 2
   ctggggtcag cacagacctg ccggcttcgc ttgg 34
<210> 3
   <211> 35
   <212> DNA
   <213> Artificial Sequence
<220>
   <223> Homo sapiens sequence bound to Brevibacillus choshinensis sequenc e
<400> 3
   catgccatgg ctttcgcttc acttgctgct gacac 35
<210> 4
   <211> 27
   <212> DNA
   <213> Homo sapiens
<400> 4
   cgcggatcct caggcactca gctctag 27